# EUROPÄISCHE PATENTSCHRIFT

(11) **EP 1 178 044 B1**
(45) Veröffentlichungstag und Bekanntmachung des Hinweises auf die Patenterteilung: **01.10.2003**
(21) Anmeldenummer: 00810703.9
(22) Anmeldetag: 04.08.2000
(51) Int. Cl.: C07D 311/72, C08F 283/06, A61K 31/355, A61P 17/00, A61K 7/06

(54) **Tocopherylalkoxylate, deren Herstellung und Verwendung in kosmetischen und pharmazeutischen Formulierungen**
Tocopherylalkoxylates, their preparation and their use in cosmetic and pharmaceutical forms
Tocophérylalkoxylates, leur préparation et leur usage dans des formulations cosmétiques et pharmaceutiques

(43) Veröffentlichungstag der Anmeldung: 06.02.2002
(73) Patentinhaber: Dr. W. Kolb AG, 8908 Hedingen (CH)
(72) Erfinder: Nägeli, Ivo, 6300 Zug (CH); Fraeys de Veubeke, Benoit, 8906 Bonstetten (CH); Kropf, Urs, 8910 Affoltern a. Albis (CH); Meyer, Marie-Sophie, 8906 Bonstetten (CH); Möhle, Horst Lothar, 8907 Wettswil (CH)
(74) Vertreter: Isler & Pedrazzini AG

(56) Entgegenhaltungen:
- WO-A-99/62896
- US-A- 5 235 073

## Beschreibung

Die Erfindung betrifft neue, oberflächenaktive Stoffe, sowie deren Herstellung und Verwendung im Bereich der Kosmetik und Pharmazeutik. Insbesondere betrifft die Erfindung oberflächenaktive, nichtionische Tenside auf der Basis von alkoxyliertem Vitamin E, die zusätzlich zu den oberflächenaktiven weitere, sich für die Haut positiv auswirkende Eigenschaften besitzen.

Um hydrophobe Öle in wässrige Systeme einarbeiten zu können, werden grenzflächenaktive Stoffe, sogenannte Tenside bzw. Emulgatoren verwendet. Durch den Einsatz von Emulgatoren wird die Grenzflächenspannung zwischen den Phasen herabgesetzt, was dem irreversiblen Zusammenfliessen der dispergierten Phase entgegenwirkt und damit die Stabilität der Emulsion erhöht.

Für kosmetische und pharmazeutische Formulierungen werden häufig nichtionische Tenside verwendet. Diese werden üblicherweise durch Ethoxylierung von Fettsäuren, Fettalkoholen, Fettaminen, aber auch natürlichen Fetten und Ölen, Polyolestern, etc. mit Ethylenoxid unter Einsatz von sauren oder basischen Katalysatoren hergestellt (M. J. Schick, *Nonionic Surfactants*, Marcel Dekker, New York, 1966; N. M. van Os, *Nonionic Surfactants, Organic Chemistry*, Marcel Dekker, New York, 1998). Die auf diese Weise produzierten Emulgatoren weisen jedoch in der Regel neben den tensidischen keine sich für die Haut positiv auswirkende Eigenschaften auf.

Neben den synthetisch hergestellten Tensiden existieren auch natürlich vorkommende, amphiphile Verbindungen wie Glycolipide, Proteine, Phospholipide, Saponine, etc. Diese Biotenside zeichnen sich dadurch aus, dass sie zusätzlich zu den amphiphilen Eigenschaften auch pysiologische Funktionen im Körper übernehmen. In den vergangenen Jahren wurden verschiedene Versuche unternommen, physiologisch aktive Verbindungen derart zu modifizieren, dass grenzflächenaktive Stoffe entstehen, ohne dass der physiologische Effekt verloren geht. Dazu können auch die zahlreich synthetisierten Vitamin E-Derivate gezählt werden, die neben tensidischen unter anderem auch antioxidative Eigenschaften aufweisen. Durch Ethoxylierung von Tocopherol wurden Tocopherylether hergestellt (US 5235073). Deren Anwendung (US 5441725) für stabile Emulsionen ist jedoch aufgrund der mässigen Tensideigenschaften begrenzt. Modifizierungen dieser Ethoxylate (US 5869703 und WO 9962896) konnten die gewünschten Eigenschaften nur im begrenztem Masse lösen. Tocopherylglycoside (EP 726273) besitzen tensidische Eigenschaften, haben aber den Nachteil, dass sie nur über einen relativ aufwendigen Syntheseweg zugänglich sind.

Es war eine Aufgabe der vorliegenden Erfindung, Tocopherylderivate herzustellen, die einerseits leicht zugänglich sind, andererseits gute Emulgiereigenschaften aufweisen und zusätzlich weitere, sich für die Haut positiv auswirkende Eigenschaften besitzen. Ferner sollten solche Derivate durch eine einfache Modifikation der Struktur den jeweiligen kolloidchemischen Anforderungen angepasst werden können.

Es war überraschend und vom Fachmann nicht vorauszusehen, dass Tocopherylalkoxylate der allgemeinen Formel (I) 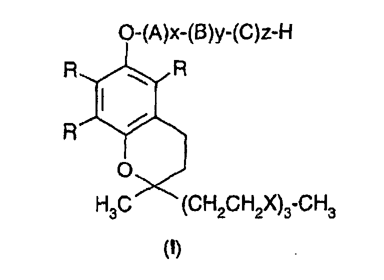 wobei x Werte von 0 bis 100 insbesondere von 0 bis 10, y Werte von 0 bis 100, insbesondere 0 bis 20 und z Werte von 1 bis 100, insbesondere 0 bis 20 einnehmen kann, dabei aber die Summe von x und y stets grösser als 0 ist, A = -CH₂CH(CH₂CH₃)O-, B = -CH₂CH(CH₃)O- und C = -CH₂CH₂O- ist, R stellvertretend für die Substituenten H- und CH₃- steht und X entweder -CH₂CH(CH₃)- oder -CH=C(CH₃)-entspricht, d. h. die den erfindungsgemässen Tocopherylalkoxylaten zugrundeliegende Tocopheroleinheit aus allen natürlichen oder synthetisch zugängigen Tocopherolkörpern bestehen kann, exzellente Emulgiereigenschaften aufweisen, einfach zugänglich sind, sich für die Haut positiv auswirkende Eigenschaften besitzen und somit die geschilderten Nachteile des Standes der Technik beseitigen. Besonders überraschend war, dass die Sequenz der Alkoxylreste einen derart grossen und entscheidenden Einfluss auf die Grenzflächenspannung ausübt. Durch das Einschieben von lipophilen Alkoxylaten zwischen die Tocopheroleinheit und der Polyethylenglykolkette konnten unerwarteterweise die Emulgiereigenschaften eindrücklich verbessert werden. Die Möglichkeit, die Grösse und Art der dazwischengeschobenen Einheit zu variieren, erlaubt schliesslich, die erfindungsgemässen Tocopherylalkoxylaten für die jeweiligen kolloidchemischen Applikationen anzupassen und zu optimieren.

Ein bevorzugtes Verfahren zur Herstellung der erfindungsgemässen Tocopherylalkoxylate ist dadurch gekennzeichnet, dass Tocopherol in Gegenwart eines basischen Katalysators, insbesondere KOH mit einer Konzentration zwischen 0.1 % und 7 %, insbesondere zwischen 0.5 % und 2 % erst mit einem Alkylenoxid, insbesondere Propylenoxid oder Butylenoxid, dann mit einem dem erstgenannten Alkylenoxid verschiedenen Alkylenoxid, insbesondere Ethylenoxid oder Propylenoxid und schliesslich, falls in der vorangegangenen Phase nicht bereits Ethylenoxid eingesetzt wurde, mit Ethylenoxid umgesetzt wird. Dabei wird die Reaktionstemperatur vorteilhaft zwischen 50 °C und 180 °C, insbesondere zwischen 80 °C und 150 °C und der Druck vorteilhaft zwischen 0.1 bar und 4 bar, insbesondere zwischen 0.5 bar und 2 bar gehalten. Es ist von Vorteil, die vorstehende Reaktion unter zumindest weitgehendem, insbesondere vollständigem Wasserausschluss durchzuführen.

Es war für den Fachmann nicht vorauszusehen, dass die erfindungsgemässen Tocopherylalkoxylate bzw. die damit hergestellten kosmetischen und/oder pharmazeutischen Zubereitungen nach der Applikation die Hautfeuchtigkeit erhöhen und zusätzlich zumindest ein Teil der dem Vitamin E eigenen antioxidierenden Eigenschaften aufweisen und dadurch sich für die Haut positiv auswirkende Eigenschaften besitzen.

Die erfindungsgemässen Formulierungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0.001 % bis 50 %, insbesondere aber 0.01 % bis 20 %, bezogen auf das Gesamtgewicht des Mittels, an den erfindungsgemässen Tocopherylalkoxylaten.

Zur Anwendung werden die erfindungsgemässen kosmetischen und/oder pharmazeutischen Zubereitungen in der für Kosmetika und/oder Pharmaka üblichen Weise auf die Haut und/oder die Haare in ausreichender Menge appliziert. Die erfindungsgemässen Formulierungen können in verschiedenen Formen vorliegen. So können sie z. B. eine Lösung, eine wasserfreie Zubereitung, eine Emulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen.

Die nachfolgenden Beispiele sollen die vorliegende Erfindung verdeutlichen, jedoch ohne sie einzuschränken.

### Beispiel 1: Herstellung des Polyethylenpolypropylen-Tocopherol-2-PO-14-EO

In einem 2 I Glas-Doppelmantel-Autoklaven wurden 276 g (0.64 mol) DL-α-Tocopherol und 2.66 g (24 mmol) einer 50%igen wässrigen KOH-Lösung vorgelegt und unter Vakuum 2 Stunden bei 80 °C gerührt. Anschliessend wurden bei 130 °C gesamthaft 74 g (1.28 mol) Propylenoxid derart zugegeben, dass der Druck im Reaktor stets zwischen 1 bar und 2 bar gehalten werden konnte. Nach erfolgter Reaktion wurden 395 g (8.96 mol) Ethylenoxid bei 140 °C ebenfalls derart zugegeben, so dass der Druck im Reaktor nie über 2 bar stieg. Nachdem die Reaktion beendet war, wurde die Schmelze abgekühlt und mit Milchsäure neutralisiert. Das feste, leicht gelbliche Produkt konnte ohne zusätzliche Reinigung isoliert und für die entsprechenden Applikationen eingesetzt werden.

### Beispiel 2: Herstellung des Polyethylenpolypropylen-Tocopherol-2-PO-18-EO

In einem 2 I Glas-Doppelmantel-Autoklaven wurden 276 g (0.64 mol) DL-α-Tocopherol und 2.66 g (24 mmol) einer 50%igen wässrigen KOH-Lösung vorgelegt und unter Vakuum 2 Stunden bei 80 °C gerührt. Anschliessend wurden bei 130 °C gesamthaft 74 g (1.28 mol) Propylenoxid derart zugegeben, dass der Druck im Reaktor stets zwischen 1 bar und 2 bar gehalten werden konnte. Nach erfolgter Reaktion wurden 505 g (11.52 mol) Ethylenoxid bei 140 °C ebenfalls derart zugegeben, so dass der Druck im Reaktor nie über 2 bar stieg. Nachdem die Reaktion beendet war, wurde die Schmelze abgekühlt und mit Milchsäure neutralisiert. Das viskose, leicht gelbliche Produkt konnte ohne zusätzliche Reinigung isoliert und für die entsprechenden Applikationen eingesetzt werden. OH-Zahl: 42 mg KOH / g; Polyethylenglykol-Gehalt: 2.7 %

### Beispiel 3: Herstellung des Polyethylenpolypropylen-Tocopherol-5-PO-8-EO

In einem 2 I Glas-Doppelmantel-Autoklaven wurden 276 g (0.64 mol) DL-α-Tocopherol und 2.66 g (24 mmol) einer 50%igen wässrigen KOH-Lösung vorgelegt und unter Vakuum 2 Stunden bei 80 °C gerührt. Anschliessend wurden bei 130 °C gesamthaft 186 g (3.20 mol) Propylenoxid derart zugegeben, dass der Druck im Reaktor stets zwischen 1 bar und 2 bar gehalten werden konnte. Nach erfolgter Reaktion wurden 226 g (5.12 mol) Ethylenoxid bei 140 °C ebenfalls derart zugegeben, so dass der Druck im Reaktor nie über 2 bar stieg. Nachdem die Reaktion beendet war, wurde die Schmelze abgekühlt und mit Milchsäure neutralisiert. Das flüssige, leicht gelbliche Produkt konnte ohne zusätzliche Reinigung isoliert und für die entsprechenden Applikationen eingesetzt werden.

### Beispiel 4: Herstellung des Polyethylenpolypropylen-Tocopherol-20-PO-8-EO

In einem 2 I Glas-Doppelmantel-Autoklaven wurden 276 g (0.64 mol) DL-α-Tocopherol und 2.66 g (24 mmol) einer 50%igen wässrigen KOH-Lösung vorgelegt und unter Vakuum 2 Stunden bei 80 °C gerührt. Anschliessend wurden bei 130 °C gesamthaft 743 g (12.80 mol) Propylenoxid derart zugegeben, dass der Druck im Reaktor stets zwischen 1 bar und 2 bar gehalten werden konnte. Nach erfolgter Reaktion wurden 226 g (5.12 mol) Ethylenoxid bei 140 °C ebenfalls derart zugegeben, so dass der Druck im Reaktor nie über 2 bar stieg. Nachdem die Reaktion beendet war, wurde die Schmelze abgekühlt und mit Milchsäure neutralisiert. Das flüssige, leicht gelbliche Produkt konnte ohne zusätzliche Reinigung isoliert und für die entsprechenden Applikationen eingesetzt werden.

### Beispiel 5: Herstellung des Polyethylenpolypropylen-Tocopherol-20-PO-10-EO

In einem 2 I Glas-Doppelmantel-Autoklaven wurden 276 g (0.64 mol) DL-α-Tocopherol und 2.66 g (24 mmol) einer 50%igen wässrigen KOH-Lösung vorgelegt und unter Vakuum 2 Stunden bei 80 °C gerührt. Anschliessend wurden bei 130 °C gesamthaft 743 g (12.80 mol) Propylenoxid derart zugegeben, dass der Druck im Reaktor stets zwischen 1 bar und 2 bar gehalten werden konnte. Nach erfolgter Reaktion wurden 282 g (6.40 mol) Ethylenoxid bei 140 °C ebenfalls derart zugegeben, so dass der Druck im Reaktor nie über 2 bar stieg. Nachdem die Reaktion beendet war, wurde die Schmelze abgekühlt und mit Milchsäure neutralisiert. Das flüssige, leicht gelbliche Produkt konnte ohne zusätzliche Reinigung isoliert und für die entsprechenden Applikationen eingesetzt werden.

### Beispiel 6: Herstellung des Polyethylenpolybutylen-Tocopherol-2-BuO-15-EO

In einem 2 l Glas-Doppelmantel-Autoklaven wurden 276 g (0.64 mol) DL-α-Tocopherol und 2.66 g (24 mmol) einer 50%igen wässrigen KOH-Lösung vorgelegt und unter Vakuum 2 Stunden bei 80 °C gerührt. Anschliessend wurden bei 130 °C gesamthaft 92 g (1.28 mol) 1,2-Butylenoxid derart zugegeben, dass der Druck im Reaktor stets zwischen 1 bar und 2 bar gehalten werden konnte. Nach erfolgter Reaktion wurden 423 g (9.60 mol) Ethylenoxid bei 140 °C ebenfalls derart zugegeben, so dass der Druck im Reaktor nie über 2 bar stieg. Nachdem die Reaktion beendet war, wurde die Schmelze abgekühlt und mit Milchsäure neutralisiert. Das flüssige, leicht gelbliche Produkt konnte ohne zusätzliche Reinigung isoliert und für die entsprechenden Applikationen eingesetzt werden.

### Beispiel 7: Herstellung des Polyethylenpolypropylenpolybutylen-Tocopherol-2-BuO-2-PO-15-EO

In einem 2 I Glas-Doppelmantel-Autoklaven wurden 276 g (0.64 mol) DL-α-Tocopherol und 2.66 g (24 mmol) einer 50%igen wässrigen KOH-Lösung vorgelegt und unter Vakuum 2 Stunden bei 80 °C gerührt. Anschliessend wurden bei 130 °C gesamthaft 92 g (1.28 mol) 1,2-Butylenoxid, dann 74 g (1.28 mol) Propylenoxid derart zugegeben, dass der Druck im Reaktor stets zwischen 1 bar und 2 bar gehalten werden konnte. Nach erfolgter Reaktion wurden 423 g (9.60 mol) Ethylenoxid bei 140 °C ebenfalls derart zugegeben, so dass der Druck im Reaktor nie über 2 bar stieg. Nachdem die Reaktion beendet war, wurde die Schmelze abgekühlt und mit Milchsäure neutralisiert. Das flüssige, leicht gelbliche Produkt konnte ohne zusätzliche Reinigung isoliert und für die entsprechenden Applikationen eingesetzt werden.

### Beispiel 8: Vergleich der Oberflächenspannung

Die Oberflächenspannung der Produkte, ein Mass für die Emulgierfähigkeit der Tenside, wurde mit einem Prozessortensiometer K12 von Krüss nach DIN 53914 ermittelt. Das jeweilige Produkt wurde als 0.1%ige wässrige Lösung gemessen. Die für den Vergleich mit dem Stand der Technik herangezogenen Werte stammen aus WO 9962896.

| | Oberflächenspannung (mN/m) |
|---|---|
| Verbindung aus Beispiel 1 | 46 |
| Verbindung aus Beispiel 2 | 31 |
| Verbindung aus Beispiel 4 | 46 |
| Verbindung aus Beispiel 5 | 37 |
| Verbindung aus Beispiel 6 | 43* |
| Verbindung aus Beispiel 7 | 44* |
| Tocopherol-12-EO | 56.3 |
| Tocopherol-10-EO-2-PO | 49.8 |

| | |
|---|---|
| * Konzentration der gemessenen Lösung: 0.01% | |

### Beispiel 9: Formulierung einfacher Emulsionen mit Hilfe der erfindungsgemässen Emulgatoren

Um die Wirksamkeit und die Anpassungsfähigkeit der erfindungsgemässen Emulgatoren zu demonstrieren, wurden einfache Emulsionen, d. h. ohne Zuhilfenahme von Hilfsstoffen, hergestellt und deren Stabilität geprüft. Die Emulsionen wurden folgendermassen Hergestellt:
O/W-Emulsionen: Eine Lösung von 4 g Emulgator in 20 g Öl wurden auf 80 °C erwärmt und zu 76 g Wasser (80 °C) gegeben, homogenisiert und anschliessend abgekühlt. W/O-Emulsionen: Eine Lösung von 7 g Emulgator in 63 g Wasser wurden auf 80 °C erwärmt und zu 30 g Öl (80 °C) gegeben, homogenisiert und anschliessend abgekühlt.

| Öl | Emulgator | Emulsionstyp | Stabilität (Tage) |
|---|---|---|---|
| Paraffinöl / Isopropylmyristat 1:1 | Beispiel 1 | O/W | > 120 |
| Paraffinöl / Isopropylmyristat 1:1 | Beispiel 2 | O/W | > 120 |
| Avocadoöl / Jojobaöl / Mandelöl 1:1:1 | Beispiel 3 | W/O | > 120 |
| C₈C₁₀-Triglycerid / Isopropylpalmitat / Octyldodecanol 1:1:1 | Beispiel 4 | W/O | > 120 |
| Paraffinöl / Isopropylmyristat 1:1 | Beispiel 5 | W/O | 25 |

### Beispiel 10: Formulierung einer Sonnenschutzcreme

| Material (INCl) | Gehalt (%) |
|---|---|
| Produkt aus Beispiel 1 | 5.00 |
| Stearic Acid | 4.00 |
| Cetearyl Alcohol | 1.80 |
| Octyldodecanol | 9.00 |
| Caprylic/Capric Triglyceride | 11.00 |
| Buxus chinensis | 3.50 |
| Butyl Methoxydibenzoylmethane | 1.00 |
| Octyl Methoxycinnamate | 5.00 |
| Aqua | auf 100 |
| Glycerin | 3.00 |

### Beispiel 11: Formulierung einer kosmetischen Lotion

| Material (INCl) | Gehalt (%) |
|---|---|
| Produkt aus Beispiel 2 | 4.00 |
| Persea gratissima | 3.00 |
| Octyldodecanol | 5.00 |
| Isopropyl Palmitate | 8.00 |
| Oleyl Oleate | 2.00 |
| Cetearyl Alcohol | 1.00 |
| Aqua | auf 100 |
| Glycerin | 2.00 |
| Propylene Glycol | 1.00 |
| Collagen | 2.00 |
| Carbomer | 0.20 |
| Aqua | 9.80 |
| Triethanolamine | auf pH 6,5 - 7,0 |

### Beispiel 12: Formulierung eines Shampoos

| Material (INCl) | Gehalt (%) |
|---|---|
| Sodium Laureth Sulfate | 35.00 |
| Cocamidopropyl Betaine | 3.00 |
| Produkt aus Beispiel 4 | 4.00 |
| Cocamidopropyl Betaine (und) Glycol Distearate (und) Cocamide MEA (und) Cocamide DEA | 2.00 |
| Aloe barbadensis | 1.00 |
| Sodium Chloride | 2.50 |
| Aqua | auf 100 |

## Patentansprüche

1. Polyalkoxylierte Tocopherolderivate der allgemeinen Formel (I) 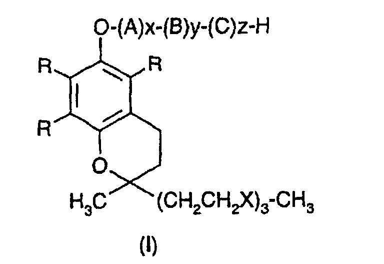 mit
0 ≤ x ≤ 100; 0 ≤ y ≤ 100; 1 ≤ z ≤ 100; x + y > 0
A = -CH₂CH(CH₂CH₃)O-; B = -CH₂CH(CH₃)O-; C = -CH₂CH₂O-;
R = -H, -CH₃
X = -CH₂CH(CH₃)-, -CH=C(CH₃)-

2. Verfahren zur Herstellung von Tocopherylalkoxylaten nach Anspruch 1, **dadurch gekennzeichnet, dass** Tocopherol der allgemeinen Formel (II) 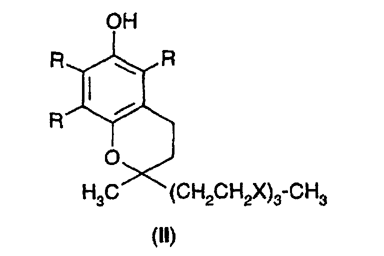 mit
R = -H, -CH₃
X = -CH₂CH(CH₃)-, -CH=C(CH₃)-
in Gegenwart eines basischen Katalysators erst mit einem Alkylenoxid, insbesondere Propylenoxid oder Butylenoxid, dann mit einem dem erstgenannten Alkylenoxid verschiedenen Alkylenoxid, insbesondere Ethylenoxid oder Propylenoxid und schliesslich, falls in der vorangegangenen Phase nicht bereits Ethylenoxid eingesetzt wurde, mit Ethylenoxid umgesetzt wird.

3. Verfahren nach Anspruch 2, **dadurch gekennzeichnet, dass** den erfindungsgemässen Tocopherylalkoxylaten zugrundeliegende Tocopheroleinheit aus allen natürlichen oder synthetisch zugängigen Tocopherolkörpern besteht.

4. Verfahren nach Anspruch 2, **dadurch gekennzeichnet, dass** der basische Katalysator KOH ist und in einer Konzentration zwischen 0.1 % und 7 % eingesetzt wird.

5. Verfahren nach Anspruch 2, **dadurch gekennzeichnet, dass** die Reaktionstemperatur zwischen 50 °C und 180 °C gehalten wird.

6. Verfahren nach Anspruch 2, **dadurch gekennzeichnet, dass** der Druck zwischen 0.1 bar und 4 bar gehalten wird.

7. Polyalkoxyliertes Tocopherolderivat gemäss Anspruch 1 zur Verwendung in kosmetischen und pharmazeutischen Formulierungen.

8. Kosmetische und pharmazeutische Formulierungen gemäss Anspruch 7, die Tocopherylalkoxylate gemäss Anspruch 1 in einer Konzentration zwischen 0.001 % und 50 % enthalten.

9. Kosmetische und pharmazeutische Formulierungen gemäss Anspruch 7, **dadurch gekennzeichnet, dass** sie eine Lösung, eine wasserfreie Zubereitung, eine Emulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen.

## Claims

1. Polyalkoxylated tocopherol derivatives of the general formula (I) 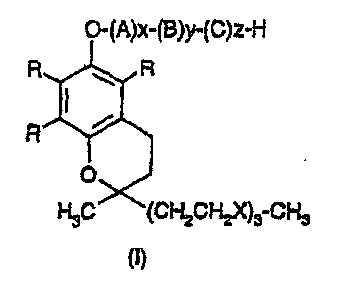 where
0 ≤ x ≤ 100; 0 ≤ y ≤100; 1 ≤ z ≤ 100; x + y > 0
A = -CH₂CH(CH₂CH₃)O-; B = -CH₂CH(CH₃)O-; C = -CH₂CH₂O-; R = -H, -CH₃
X = -CH₂CH(CH₃)-, -CH=C(CH₃)-.

2. Process for the preparation of tocopheryl alkoxylates according to Claim 1, **characterized in that** tocopherol of the general formula (II) 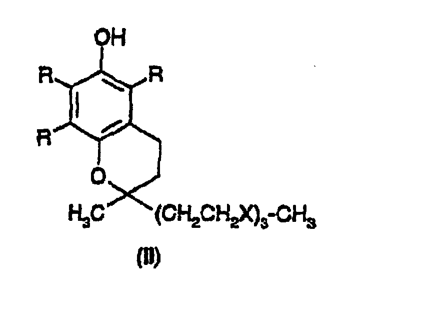 where
R = -H, -CH₃
X = -CH₂CH(CH₃)-, -CH=C(CH₃)-
is reacted in the presence of a basic catalyst firstly with an alkylene oxide, in particular propylene oxide or butylene oxide, then with an alkylene oxide different from the first-mentioned alkylene oxide, in particular ethylene oxide or propylene oxide and finally, if ethylene oxide has not already been used in the preceding phase, with ethylene oxide.

3. Process according to Claim 2, **characterized in that** the tocopherol unit which forms the basis of the tocopheryl alkoxylates according to the invention consists of all natural or synthetically accessible tocopherol bodies.

4. Process according to Claim 2, **characterized in that** the basic catalyst is KOH and is used in a concentration between 0.1% and 7%.

5. Process according to Claim 2, **characterized in that** the reaction temperature is maintained between 50°C and 180°C.

6. Process according to Claim 2, **characterized in that** the pressure is maintained between 0.1 bar and 4 bar.

7. Polyalkoxylated tocopherol derivative according to Claim 1 for use in cosmetic and pharmaceutical formulations.

8. Cosmetic and pharmaceutical formulations according to Claim 7, which comprise tocopheryl alkoxylates according to Claim 1 in a concentration between 0.001% and 50%.

9. Cosmetic and pharmaceutical formulations according to Claim 7, **characterized in that** they represent a solution, an anhydrous preparation, an emulsion of the water-in-oil (W/O) type or of the oil-in-water (O/W) type, a multiple emulsion, for example of the water-in-oil-in-water (W/O/W) type, a gel, a solid stick, an ointment or an aerosol.

## Revendications

1. Dérivés polyalcoxylés de tocophérol de formule générale (I) 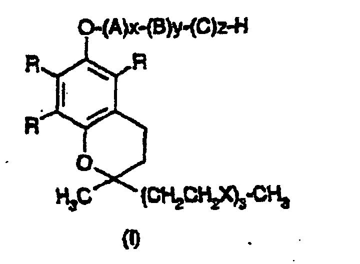 dans laquelle
0 ≤ x ≤ 100, 0 ≤ y ≤ 100, 1 ≤ z ≤ 100, x + y > 0,
A = -CH₂CH(CH₂CH₃)O-, B = -CH₂CH(CH₃)O-, C = -CH₂CH₂O-,
R = -H, -CH₃,
X = -CH₂CH(CH₃)-, -CH=C(CH₃)-.

2. Procédé de préparation de tocophérylalcoxylates selon la revendication 1, **caractérisé en ce que** l'on fait réagir du tocophérol de formule générale (II) 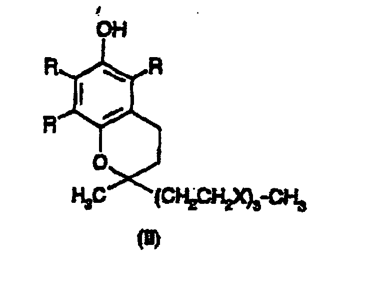 dans laquelle
R = -H, -CH₃,
X = -CH₂CH(CH₃)-, -CH=C(CH₃)-
en présence d'un catalyseur basique, d'abord avec un oxyde d'alkylène, en particulier de l'oxyde de propylène ou de l'oxyde de butylène, puis avec un oxyde d'alkylène différent de l'oxyde d'alkylène susmentionné, en particulier de l'oxyde d'éthylène ou de l'oxyde de propylène, et enfin, si l'on n'a pas déjà mis en oeuvre de l'oxyde d'éthylène dans la phase précédente, avec de l'oxyde d'éthylène.

3. Procédé selon la revendication 2, **caractérisé en ce que** l'unité tocophérol à la base des tocophérylalcoxylates selon l'invention consiste en tous les corps de tocophérol accessibles par voie naturelle ou synthétique.

4. Procédé selon la revendication 2, **caractérisé en ce que** le catalyseur basique est du KOH et est mis en oeuvre à une concentration de 0,1% à 7%.

5. Procédé selon la revendication 2, **caractérisé en ce que** la température de réaction est maintenue entre 50°C et 180°C.

6. Procédé selon la revendication 2, **caractérisé en ce que** la pression est maintenue entre 0,1 bar et 4 bars.

7. Dérivé polyalcoxylé de tocophérol selon la revendication 1 pour utilisation dans des formulations cosmétiques et pharmaceutiques.

8. Formulations cosmétiques et pharmaceutiques selon la revendication 7, contenant les tocophérylalcoxylates selon la revendication 1 en une concentration de 0,001% à 50%.

9. Formulations cosmétiques et pharmaceutiques selon la revendication 7, **caractérisées en ce qu'**elles sont sous forme d'une solution, d'une préparation anhydre, d'une émulsion du type eau dans l'huile (E/H) ou du type huile dans l'eau (H/E), d'une émulsion multiple, par exemple du type eau dans l'huile dans l'eau (E/H/E), d'un gel, d'un bâton solide, d'une pommade ou également d'un aérosol.
